# EUROPEAN PATENT SPECIFICATION

(11) **EP 0 307 437 B1**
(45) Date of publication and mention of the grant of the patent: **02.03.1994**
(21) Application number: 88902748.8
(22) Date of filing: 07.03.1988
(51) Int. Cl.: A61B 17/04, C08F 14/18

(54) **PIEZOLECTRIC NERVE GUIDANCE CHANNELS**
PIEZOELEKTRISCHE NERVENFÜHRUNGSKANÄLE
CANAUX PIEZOELECTRIQUES DE GUIDAGE DE NERFS

(30) Priority: 13.03.1987 US 25529
(43) Date of publication of application: 22.03.1989
(73) Proprietor: Brown University Research Foundation, Inc., Providence Rhode Island 02912 (US)
(72) Inventor: AEBISCHER, Patrick, Providence, RI 02960 (US); VALENTINI, Robert, F., Providence, RI 02906 (US); GALLETTI, Pierre, M., Providence, RI 02906 (US)
(74) Representative: Harvey, David Gareth
(86) International application number: US8800693
(87) International publication number: WO8806866

(56) References cited:
- EP-A- 0 286 284
- US-A- 3 786 817
- US-A- 3 833 002
- US-A- 3 916 905
- US-A- 3 925 339
- US-A- 4 074 366
- US-A- 4 268 653
- US-A- 4 534 349
- US-A- 4 668 449
- A.S.A.I.O. TRANSACTIONS, vol. 33, no. 3, 1987, pages 456-458, Hagerstown, MD, US; P. AEBISCHER et al.: "Piezoelectric nerve guidance channels enhance peripheral nerve regeneration"
- SOCIETY FOR NEUROSCIENCE ABSTRACTS, vol. 12, 1986, page 13, abstract no. 7.11; J.M. KERNS et al.: "D.c. electrical fields promote regeneration in the rat sciatic nerve after axotomy"
- American Society for Artificial Internal Organs Journal, vol.6, no.1, published 1983 (J.B.Lippincott Co. US), "The Electromechanical Connection: Piezoelectric Polymers in Artificial Organs" pages1-11
- Journal of Neuropathology and Experimental Neurology, vol.41, no.4, published July 1982, "In Vivo Regeneration of Cut Nerves Encased in Silicone Tubes" pages 412-422
- Journal of Neuroscience Research, vol.9, published 1983 "Mouse Sciatic Nerve Regeneration through Semipermeable Tubes: a Quantitative Model" pages 325-338
- Mechanisms of Growth Control, Chapter II, Fukada Echi, Springfield MD, pp 192-210
- Journal of Plastic and Reconstructive Surgery, vol.74, no.2, published 8 August 1983, "Nerve Regeneration through Synthetic Biodegradable Nerve Guides: Regulation by the Target Organ" pages 173-181
- Brain Research, vol.342, published 1985 (Elsevier Science Publishers BV) "An In Vivo Model to Quantify Motor and Sensory Peripheral Nerve Regeneration using Bioresorbable Nerve Guide Tubes" pages 307-315

## Description

The technical field of this invention concerns medical devices useful for the repair of injured nerves

The problem of repairing severed nerves is a long-standing one that has plagued surgeons for over a hundred years. Despite advances in microsurgical techniques, a patient's recovery from a serious wound is often limited by a degree of nerve damage which cannot be repaired. The replanting of amputated fingers and limbs is especially limited by poor nerve regeneration.

When a nerve is severed, the functions supplied by that nerve, both motor and sensory, are lost. The nerve cells' appendages (axons) in the distal (the furthest away from the spinal cord) portions of the severed nerve degenerate and die leaving only the sheaths in which they were contained. The axons in the proximal stump that are still connected to the spinal cord or dorsal root ganglion, also suffer some degeneration. The degeneration generally does not proceed to the death of the entire nerve cell bodies. If the injury occurs far enough from the nerve cell bodies, regeneration will occur. Axonal sprouts will appear from the tip of the regenerating axon. These sprouts grow distally and attempt to reenter the intact neurilemmal sheaths of the distal portion of the severed nerve. If entry is successfully made, axonal growth will continue down these sheaths and function will eventually be restored.

In the conventional approach to nerve repair, an attempt is made to align the cut ends of the fascicles (nerve bundles within the nerve trunk). A similar approach is taken with smaller nerves. In either case, the chief hazard to the successful repair is the trauma produced by the manipulation of the nerve ends and the subsequent suturing to maintain alignment. The trauma appears to stimulate the growth and/or migration of fibroblasts and other scar-forming connective tissue cells. The scar tissue prevents the regenerating axons in the proximal stump from reaching the distal stump to reestablish a nerve charge pathway. The result is a permanent loss of sensory or motor function.

Various attempts have been made over the years to find a replacement for direct (i.e., nerve stump-to-nerve-stump suturing). Much of the research in this field has focused on the use of "channels" or tubular prostheses which permit the cut ends of the nerve to be gently drawn into proximity and secured in place without undue trauma. It is also generally believed that such channels can also prevent, or at least retard, the infiltration of scar-forming connective tissue.

The use of silastic cuffs for peripheral nerve repair was reported by Ducker et al. in Vol. 28, Journal of Neurosurgery, pp. 582-587 (1968). Silicone rubber sheathing for nerve repair was reported by Midgley et al. in Vol. 19, Surgical Forum, pp. 519-528 (1968) and by Lundborg, et al. in Vol. 41, Journal of Neuropathology in Experimental Neurology, pp. 412-422 (1982). The use of bioresorbable polyglactin mesh tubing was reported by Molander et al. in Vol. 5, Muscle & Nerve, pp. 54-58 (1982). The use of semipermeable acrylic copolymer tubes in nerve regeneration was disclosed by Uzman et al. in Vol. 9, Journal of Neuroscience Research, pp. 325-338 (1983). Bioresorbable nerve guidance channels of polyesters and other polymers have been reported by Nyilas et al. in Vol. 29, Transactions Am. Soc. Artif. Internal Organs, pp. 307-313 (1983) and in U.S. Patent 4,534,349 issued to Barrows in 1985.

Despite the indentification of various materials which can serve as nerve guidance channels, the results of research to date have revealed significant shortcomings in such prostheses. Some of the materials identified above have lead to inflammatory reactions in the test animals and have failed to exclude scar tissue formation within the channels. Moreover, the total number of axons, the number of myelinated axons, the thickness of the epineurium, and the fascicular organization of nerves regenerated within guidance channels are all typically less than satisfactory and compare poorly with the original nerve structure of the test animals. Moreover, the loss of sensory or motor function is still the most common outcome of such laboratory experiments.

It has been suggested that nerve regeneration may be promoted by steady state d.c. electrical fields produced by battery implants and applied via wick electrodes to the nerve. Experimental work was performed on rat sciatic nerve after axotomy. See SOCIETY FOR NEUROSCIENCE ABSTRACTS, vol. 12, 1986, page 13, abstract no. 7.11; J.M. KERNS et al.: "D.c. electrical fields promote regeneration in the rat sciatic nerve after axotomy".

There exists a need for better materials for formation of nerve guidance channels. Materials for nerve repair that would minimize surgical trauma, prevent interference with nerve growth by scar tissue, and improve the chances for successful recovery of sensory or motor function, would satisfy a long-felt need in this field.

Starting from Ducker et al, Journal of Neurosurgery, Vol. 28 pp. 582-587 (1968), the present invention provides a medical device for use in regenerating a severed nerve, the device comprising a tubular membrane having openings adapted to receive the ends of the severed nerve and a lumen to permit regeneration of said nerve, characterised in that the membrane is made of a piezoelectric material.

It has been discovered that the repair of severed or avulsed nerves can be greatly enhanced by the use of piezoelectric materials as nerve guidance channels. Medical devices employing such piezoelectric materials are disclosed for use in regenerating nerves. The devices can be formed by a tubular piezoelectric conduit adapted to receive the ends of a severed or damaged nerve. The tubular membrane defines a lumen through which axons can be regenerated to restore motor and/or sensory functions. The piezoelectric materials generate transient electrical charges upon mechanical deformation which augment the ability of axons to bridge the gap between the proximal and distal stumps.

The term "piezoelectric materials" as used herein is intended to encompass natural and synthetic materials capable of generating electrical charges on their surface when subjected to mechanical strain. The preferred materials are biocompatible, semicrystalline polymers which can be poled during manufacture or prior to use in order to align the polymeric chain segments in a particular orientation and, thereby, establish a predefined dipole moment. The piezoelectric materials of the present invention are preferably poled to establish a charge generation (polarization constant) ranging from about 0.5 to about 35 picoColoumbs per Newton, and, more preferably, from about 1 to about 20 picoColoumbs per Newton.

Piezoelectric materials useful in the present invention include a variety of halogenated polymers, copolymers and polymer blends. The halogenated polymers include polyvinylidene fluoride, polyvinyl fluoride, polyvinyl chloride and derivatives thereof as well as copolymers such as copolymers of the above materials and trifluoroethylene. Non-halogenated piezoelectric polymers which may also be useful in the present invention include collagen, nylon 11, and alpha-helical polypeptides such as polyhydroxybutyrate, poly-γ-benzyl-glutamate and poly-γ-methyl-glutamate In some applications it may also be possible to use thin piezoelectric ceramics, such as barium titanate, lead titanate or lead zirconate or combinations of such ceramic and polymeric materials.

One particularly preferred piezoelectric material for nerve guidance channels is polyvinylidene fluoride ("PVDF" or "PVF₂) especially after it has been poled to impart a high polarization constant. PVDF is a semicrystalline polymer formed by the sequential addition of (CH₂-CF₂)ₙ repeat units, where n can range from about 2,000 to about 15,000. Crystallographers have described various stable forms or phases of PVDF. The alpha phase, which is generally obtained by cooling the melt at atmospheric pressure, has a monoclinic unit cell with chain segments in antipolar orientation, and thus no net dipole moment.

The beta phase of PVDF, which displays the highest piezoelectric activity, has an orthorhombic unit cell containing two chains with the same orientation, giving it a permanent dipole moment. To display its piezoelectric properties, PVDF must be anisotropic, i.e., its electrical properties must be quantitatively different for mechanical excitation along different axes. In PVDF as well as other semicrystalline polymer films, the isotropy that generally prevails can be altered by molecular orientation, usually induced by mechanical stretching, followed by alignment of the permanent dipoles in a direction perpendicular to the plane of the film by an electric field (a "poling" process).

The use of tubular nerve guidance channels of poled PVDF has been found to surpass all other materials tested to date as guidance channels. When compared with unpoled PVDF, the poled material achieved significantly better results (over twice as many myelinated axons after four weeks) as a nerve guidance material. The success of poled PVDF as a nerve guidance channel material appears to lie in its biocompatibility and high piezoelectric activity. The best results to date have been obtained with tubular PVDF which is poled to generate positive charges on the inner (luminal) surface of the tubes upon mechanical deformation.

The piezoelectric nerve guidance channels of the present invention can also be semipermeable to permit passage of nutrients and metabolites (i.e., having molecular weights of about 100,000 daltons or less) through the channel walls. The permeability can be controlled such that scar-forming cells are excluded from the lumen while growth factors released by the injured nerves are retained within the lumen. Various techniques known in the art, such as the use of degradable derivatives or the formation of copolymers having a biodegradable component can be employed to obtain a satisfactory degree of permeability in use. If the channel is not totally biodegradable over time, it can be formed with longitudinal lines of weakness to facilitate removal from about the regenerated nerve after healing has progressed sufficiently.

Preferably, the membrane wall thickness of the piezoelectric nerve guidance channels of the present invention will range from about 0.05 to about 1.0 millimeters. Similarly, the diameter of lumen can vary from about 0.5 millimeters to about 2 centimeters, depending upon the size of nerve to be repaired.

In the case of polyvinylidene fluoride (PVDF), the beta phase, which displays the greatest piezoelectric activity, can be obtained by mechanically stretching and annealing alpha phase PVDF. The stretching process orients crystalline unit cells, known as spherulites, with their long axis perpendicular to the direction of elongation. Poling the beta phase PVDF under a high electrical field freezes the random dipoles and creates a permanent, strong dipole moment. The polarity of the electrodes determines the net charge on the outer and luminal surfaces of the tube (i.e., the orientation of the dipoles). Therefore, depending on the poling procedure, tubes which generate upon mechanical deformation either positive or negative charges on their luminal surface can be fabricated. Poling procedures avoiding polymer contact, such as corona discharge, are used to prevent polymer breakdown.

The nerve guidance channels of the present invention are used by locating the severed nerve ends, and selecting an appropriately sized piezoelectric tubular device for the repair, having openings adapted to receive the ends of the severed nerve and a lumen to permit regeneration of the nerve therethrough. The cut ends of the nerve are then gently drawn into tube by manual manipulation or suction, placed in optimal proximity and then secured in position without undue trauma by sutures through the tube, or by a biocompatible adhesive (e.g., fibrin glue) or by frictional engagement with the tube. The tube is then situated such that muscle contractions and general animal movement induce mechanical deformation and, hence, generation of electrical charges within the lumen. Antibiotics can be administered to the site, and the wound is then closed.

The term "nerve" is used herein to mean both monofascicular and polyfascicular nerves . The same general principals of regeneration with piezoelectric nerve guidance channels are applicable to both.

The invention will next be described in connection with certain preferred embodiments; however, it should be clear that various changes can be made by those skilled in the art within the scope of the appended claims. For example, although the piezoelectric nerve guidance channels described below are generally tubular in shape, it should be clear that various alternative shapes can be employed: The lumens of the guidance channels can be oval or even square in cross-section. The guidance channels can also be constructed from two or more parts which are clamped together to secure the nerve stumps. Moreover, sheet piezoelectric materials can be employed and formed into tube in situ. In such a procedure, the nerve stumps can be placed on top of the sheet and secured thereto by sutures, adhesives or friction. The sheet is then wrapped around the nerve segments and the resulting tube is closed by further sutures, adhesives or friction.

The nerve guidance channels of the present invention can also take advantage of related pyroelectric properties which are often also exhibited by piezoelectric materials. Pyroelectric effects are typically defined as the exhibition of electrical polarization as a result of temperature changes. Thus, the nerve guidance channels of the present invention can also employ temperature changes to create transient electric charges on the inner surface of the lumen.

Various materials can also be used to fill the luminal cavity. For example, the cavity can be filled with physiological saline, laminin, collagen, glycosaminoglycans or nerve growth factors. The cavity can also be seeded with cultured Schwann cells.

### Brief Description of the Drawings

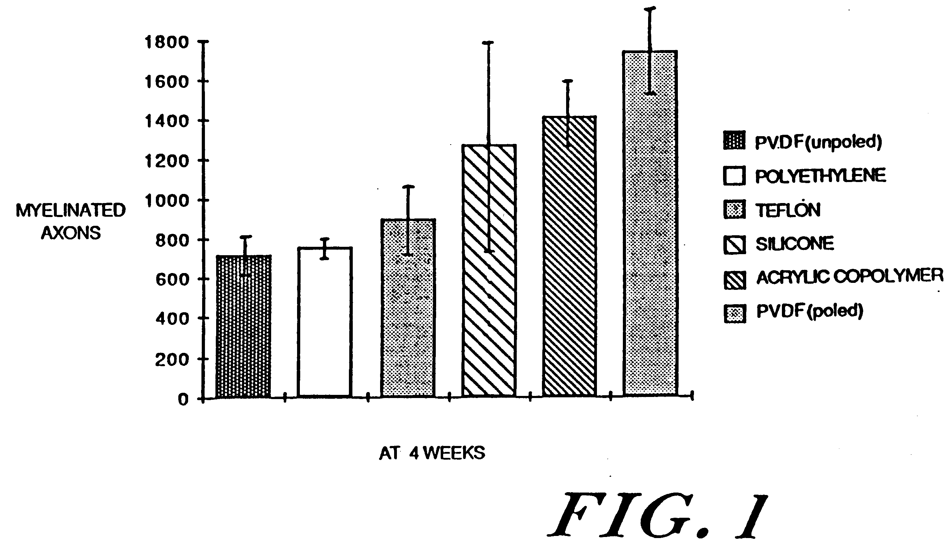

FIG. 1 is a comparative graph of the regenerative capabilities (in terms of numbers of myelinated axons) of various piezoelectric and non-piezoelectric nerve guidance materials.

### Detailed Description

The invention will next be described in connection with the following examples and comparative experiments.

Young female CD-1 mice (25-30 g) (Charles River Lab., Wilmington, MA) were housed in temperature and humidity-controlled rooms and received food and water ad libitum. The mice were anesthetized with methoxyfluorane and the left sciatic nerve was exposed through an incision along the anterior-medial aspect of the upper thigh. After retraction of the gluteus maximus muscle, a 3-4 mm segment of nerve proximal to the tibio-peroneal bifurcation was resected and discarded.

A series of materials were then tested as nerve guidance channels. The materials were all tubular in shape and 6 mm long. The nerve stumps were anchored 4 mm apart within the tubes using 10-0 nylon sutures placed through holes 1 mm from each channel end. For each material, at least six channels were implanted for a period of four weeks. A further set of control animals underwent nerve resection as described apart, and their section sites were closed without implantation of any guidance material. Aseptic surgical technique was maintained throughout the procedures, which were performed with the aid of an operating microscope.

A variety of non-piezoelectric materials were used as tubular guidance channels for comparison purposes. These non-piezoelectric materials included polyethylene [Clay Adams, Parsippany, NJ], Teflon™ [Gore, Flagstaff AZ], silicone [Silmed, Taunton, MA], and acrylic copolymer (Amicon XD-50 tubing, Lexington, Massachusetts). In addition, non-polarized PVDF was compared with identical PVDF tubing which had undergone poling.

The piezoelectric guidance materials were manufactured from pellets of homopolymeric PVDF (Solef XION, Solvay & Cie, Brussels, Belgium). The pellets were extruded into tubes with an outer diameter (OD) of 2.5 mm and an internal diameter (ID) of 1.85 mm. The extruded tubes were stretched 3.5 times along their axes at a temperature of 110°C and at a rate of 1 cm per minute. This stretching process transformed the alpha non-polar crystalline phase into the beta polar crystalline phase. The tubes were then annealed by maintaining the tension on the tubes for 3 hours at 110°C. The final OD and ID were 1.25 mm and 0.87 mm, respectively.

Some tubes were then cut and poled under an electric field to permanently orient the molecular dipoles of the beta phase. A thin wire inserted in the lumen of the stretched PVDF tubes served as an inner electrode and a circumferentially regularly oriented array of steel needles served as the outer electrode. The outer electrodes were connected to the positive output of a voltage supply (Model 205-30P, Bertran Associates Inc., Syosset, NY), and the inner electrode was grounded. The voltage was increased gradually over 2 hours until it reached 21kv and was then maintained for 12 hours. A second set of tubes was poled by connecting the positive output of the voltage supply to the inner electrode and grounding the outer electrode. In both cases, this poling procedure resulted in the generation of surface electrical charges upon mechanical deformation of the tube. Electrical charge distribution was dependent on the local mechanical strain on the tube; the pattern of electrical charges is opposite in the tubes prepared with reversed polarity.

In order to determine the piezoelectric activity of the poled tubes, their outer surface was coated with a thin layer of silver paint and the inner electrode was repositioned. A vertical deflection of 1 mm was induced in the center of each tube by a rotating cam connected to a DC micromotor. The average charge generated by the tubes poled with positive or negative external electrodes was 200-300 pC. These measurements translated into polarization constants ranging from about 10 to about 15 picoColumbs per Newton for the poled PVDF tubes. Unpoled tubes did not generate a detectable charge upon deformation.

Both poled and unpoled PVDF tubes were washed in acetone, rinsed several times with saline, and cleaned ultrasonically before being sterilized in an ethylene oxide gas chamber at 40°C.

At retrieval time, the animals were deeply anesthetized and perfused transcardially with 5 ml of phosphate-buffered saline (PBS) followed by 10 ml of a fixative containing 3.0% paraformaldehyde and 2.5% glutaraldehyde in PBS at pH 7.4. The operative site was reopened and the guidance channel and segments of the native nerve at either channel end were removed. The specimens were then post-fixed in a 1% osmium tetroxide solution, dehydrated and embedded in Spurr resin. Transverse sections taken at the midpoint of the guidance channel were cut on a Sorvall MT-5000 microtome. The sections (1 micron thick) were stained with toluidine blue. Whole mounts of nerve were displayed on a video monitor through a Zeiss IM35 microscope. Nerve cable cross-sectional area and the number of myelinated axons were determined with the aid of a graphic tablet at a final magnification of 630x. The Wilcoxon Rank-sum test was used to assess statistical differences (p < 0.05) between the various populations. All values are presented as means ± standard error of the mean.

The results of the comparative studies are shown in graphic form in FIG. 1. The number of myelinated axons found upon reexposure after four weeks for each of the tested guidance materials is shown. Peripheral nerve regeneration was dramatically enhanced by the use of piezoelectric guidance channels which generated either transient positive or negative charges on their inner surface. Nerves regenerated in poled PVDF tubes contained significantly more myelinated axons and displayed more normal morphological characteristics than nerves regenerated in unpoled tubes. When compared to all other materials tested, the poled PVDF tubes contained the highest number of myelinated axons at four weeks, and the regenerated axons displayed greater diameter and myelin sheath thickness.

Upon reexposure, all retrieved guidance channels were covered by a thin tissue layer which did not reduce the translucence of the PVDF tubes. A cable bridging the nerve stumps was observed in all implanted tubes. All cables were surrounded by an a cellular gel and were free from attachment to the guidance channel wall. In stark contrast, mice with no guidance channel showed complete nerve degeneration.

Nerve cables regenerated in poled and unpoled PVDF tubes differed considerably with respect to their cross-sectional area, relative tissue composition and number of myelinated axons. The cross-sectional area of the regenerated cables at the midpoint of the poled PVDF guidance channels were significantly larger than those regenerated in unpoled PVDF channels (4.33 ± 1.25 versus 2.35 ± 1.17 mm² x 10⁻²; p < 0.05). The cables were delineated by an epineurium composed mainly of fibroblasts and collagen fibrils which surrounded numerous fascicles containing myelinated and nonmyelinated axons and Schwann cells. The relative percentage of fascicle area was significantly greater in poled tubes whereas the relative percentage of epineurial tissue was significantly smaller (Table 1). Although the relative area of blood vessels was higher in poled tubes, the difference was not statistically significant (Table 1). Most importantly, the nerves regenerated in poled PVDF tubes contained significantly more myelinated axons than those in unpoled PVDF tubes (1,742 ± 352 versus 778 ± 328; p < than 0.005).

**TABLE 1**

| COMPARISON OF POLED AND UNPOLED PVDF NERVE GUIDANCE CHANNELS | | |
|---|---|---|
| | Poled | Unpoled |
| Myelinated Axons | 1,742 ± 352 | 778 ± 328 |
| Fascicular Area* | 71.2 ± 3.9 | 65.2 ± 5.1 |
| Epineurial Area* | 19.0 ± 4.0 | 28.0 ± 6.0 |
| Blood vessel Area* | 9.8 ± 3.4 | 6.8 ± 3.2 |

| | | |
|---|---|---|
| *Relative surface area of the different nerve cable components in percent. | | |

## Claims

1. A medical device for use in regenerating a severed nerve, the device comprising a tubular membrane having openings adapted to receive the ends of the severed nerve and a lumen to permit regeneration of said nerve, characterised in that the membrane is made of a piezoelectric material.

2. The device of claim 1 wherein the piezoelectric material has a polarization constant ranging from about 0.5 to about 35 picoColoumbs per Newton, for example from about 1 to about 20 picoCoulombs per Newton.

3. The device of claim 1, wherein the piezoelectric material comprises a material selected from the group of polyvinylidene fluoride, polyvinyl fluoride, polyvinyl chloride, collagen, nylon 11, polyhydroxybutyrate, poly-γ-benzyl-glutamate, poly-γ-methyl-glutamate, copolymers of trifluoroethylene and such polymers, and derivatives of such polymers.

4. The device of claim 1 wherein the piezoelectric material is polyvinylidene fluoride which, for example, has a chain length of about 2000 to about 15,000 repeat units.

5. The device of claim 4, wherein the polyvinylidene fluoride material exhibits a orthorhombic unit cell structure containing two chains with the same orientation and, consequently, a permanent dipole moment.

6. The device of claim 5, where the polyvinylidene fluoride material further exhibits an alignment of the permanent dipoles and a polarization constant of about 1 to about 20 picoColoumbs per Newton.

7. The device of claim 1, wherein the thickness of the membrane ranges from about 0.05 to about 1.0 millimeter.

8. The device of claim 1, wherein the membrane is permeable to solutes having a molecular weight of about 100,000 daltons or less.

9. The device of claim 1, wherein the membrane is polarized such that a positive charge is generated at the inner membrane surface upon mechanical deformation.

10. The device of claim 1, wherein the membrane is polarized such that a negative charge is generated at the inner membrane surface upon mechanical deformation.

## Patentansprüche

1. Eine medizinische Vorrichtung zur Verwendung bei der Regenerierung eines durchtrennten Nervens, wobei die Vorrichtung eine röhrchenförmige Membran umfaßt, die der Aufnahme der Enden des abgetrennten Nervens angepaßte Öffnungen und ein Lumen, das die Regenerierung dieses Nervens ermöglicht, umfaßt, dadurch gekennzeichnet, daß die Membran aus einem piezoelektrischen Material hergestellt ist.

2. Die Vorrichtung nach Anspruch 1, bei der das piezoelektrische Material eine Polarisationskonstante aufweist, die von etwa 0,5 bis etwa 35 picoCoulomb pro Newton, z.B. von etwa 1 bis etwa 20 picoCoulomb pro Newton, reicht.

3. Die Vorrichtung nach Anspruch 1, bei der das piezoelektrische Material ein Material umfaßt, das aus der Gruppe aus Polyvinylidenfluorid, Polyvinylfluorid, Polyvinylchlorid, Collagen, Nylon 11, Polyhydroxybutyrat, Poly-γ-benzyl-glutamat, Poly-γ-methyl-glutamat, Copolymeren von Trifluorethylen und solchen Polymeren und Derivaten von solchen Polymeren ausgewählt ist.

4. Die Vorrichtung nach Anspruch 1, bei der das piezoelektrische Material Polyvinylidenfluorid ist, das z.B. eine Kettenlänge von etwa 2000 bis etwa 15000 Struktureinheiten aufweist.

5. Die Vorrichtung nach Anspruch 4, bei der das Polyvinylidenfluoridmaterial eine orthorhombische Einheitenzellstruktur aufweist, die zwei Ketten mit der gleichen Orientierung und demzufolge ein permanentes Dipolmoment enthält.

6. Die Vorrichtung nach Anspruch 5, bei der das Polyvinylidenfluoridmaterial weiterhin eine Ausrichtung der permanenten Dipole und eine Polarisationskonstante von etwa 1 bis etwa 20 picoCoulomb pro Newton aufweist.

7. Die Vorrichtung nach Anspruch 1, bei der die Dicke der Membran von etwa 0,05 bis etwa 1,0 Millimeter reicht.

8. Die Vorrichtung nach Anspruch 1, bei der die Membran für gelöste Stoffe mit einem Molekulargewicht von etwa 100000 Dalton oder weniger durchlässig ist.

9. Die Vorrichtung nach Anspruch 1, bei der die Membran derart polarisiert ist, daß eine positive Ladung bei mechanischer Deformation an der inneren Membranoberfläche erzeugt wird.

10. Die Vorrichtung nach Anspruch 1, bei der die Membran derart polarisiert ist, daß eine negative Ladung bei mechanischer Deformation an der inneren Membranoberfläche erzeugt wird.

## Revendications

1. Dispositif médical utilisable dans la régénération d'un nerf sectionné, ce dispositif étant constitué par une membrane tubulaire présentant des ouvertures propres à recevoir les extrémités du nerf sectionné et une lumière permettant la régénération dudit nerf, caractérisé en ce que la membrane est faite d'une matière piézoélectrique.

2. Dispositif selon la revendication 1, dans lequel la matière piézoélectrique a une constante de polarisation dans la gamme d'environ 0.5 à environ 35 picocoulombs par newton, par example d'environ 1 à environ 20 picocoulombs par newton.

3. Dispositif selon la revendication 1, dans lequel la matière piezoéléctrique est constituée par une matière choisie dans le groupe comprenant le polyfluorure do vinylidène, le polyfluorure de vinyle, le polychlorure de vinyle, le collagène, le nylon-11, le polyhydroxybutyrate, le poly-γ-benzylglutamate, le poly-γ-méthylglutamate, des copolymères de trifluoroéthylène et de ces polymères, et des dérivés de ces polymères.

4. Dispositif selon la revendication 1, dans lequel la matière piezoéléctrique est un polyfluorure de vinylidene qui a par exemple une longueur de chaîne d'environ 2000 à environ 15 000 motifs repétitifs.

5. Dispositif selon la revendication 4, dans lequel la matière de polyfluorure de vinylidene présente une structure à mailles orthorhombiques contenant deux chaînes de même orientation et, en conséquence, un moment dipolaire permanent.

6. Dispositif selon la revendication 5, dans lequel la matière de polyfluorure de vinylidène présente en outre un alignement des dipôles permanents et une constante de polarisation d'environ 1 à environ 20 picocoulombs par newton.

7. Dispositif selon la revendication 1, dans lequel l'épaisseur de la membrane se situe dans la gamme d'environ 0.05 à environ 1,0 mm.

8. Dispositif selon la revendication 1, dans lequel la membrane est perméable aux produits dissous ayant un poids moléculaire d'environ 100 000 daltons ou moins.

9. Dispositif selon la revendication 1, dans lequel la membrane est polarisée de sorte qu'une charge positive soit créée au niveau de la surface interne de la membrane sous l'effet d'une déformation mécanique.

10. Dispositif selon la revendication 1, dans lequel la membrane est polarisée de sorte qu'une charge négative soit créée au niveau de la surface interne de la membrane sous l'effet d'une deformation mécanique.
